Europäisches Patentamt

European Patent Office

Office européen des brevets

(19)

(11) **EP 1 050 320 B1**

(12) **FASCICULE DE BREVET EUROPEEN**

(45) Date de publication et mention
de la délivrance du brevet:
**06.04.2005 Bulletin 2005/14**

(51) Int Cl.$^7$: **A61N 1/368**

(21) Numéro de dépôt: **00401262.1**

(22) Date de dépôt: **09.05.2000**

(54) **Dispositif médical implantable actif, notamment stimulateur cardiaque, défibrillateur et/ou cardioverteur de type DDD/AAI à fonctionnement optimisé à l'effort**

Aktive implantierbare medizinische Vorrichtung, insbesondere Herzstimulator, Defibrillator und/oder Kardiovertierer von Typ DDD/AAI, dessen Takt durch die Belastung des Patienten gesteuert wird

Active implantable medical device, in particular pacemaker, defibrillator and/or cardioverter of type DDD/AAI responsive to the patient effort

(84) Etats contractants désignés:
**AT BE CH CY DE DK ES FI FR GB GR IE IT LI LU MC NL PT SE**

(30) Priorité: **07.05.1999 FR 9905821**

(43) Date de publication de la demande:
**08.11.2000 Bulletin 2000/45**

(73) Titulaire: **ELA MEDICAL (Société anonyme)**
**92541 Montrouge (FR)**

(72) Inventeurs:
• **Bouhour, Anne**
**92410 Ville d'Avray (FR)**

• **Bonnet, Jean-Luc**
**92120 Montrouge (FR)**

(74) Mandataire: **Dupuis-Latour, Dominique et al**
**SEP Pagenberg & Associés**
**14 boulevard Malesherbes**
**75008 Paris (FR)**

(56) Documents cités:
**EP-A- 0 488 904**     **EP-A- 0 600 631**
**US-A- 4 945 909**     **US-A- 5 334 222**
**US-A- 5 873 895**

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, dispositifs "multisite" (triple ou quadruple chambre), défibrillateurs et/ou cardioverteurs, permettant de délivrer au coeur des impulsions électriques de faible énergie pour le traitement des troubles du rythme cardiaque.

Elle concerne plus particulièrement ceux de ces dispositifs qui comprennent des circuits de stimulation à commutation automatique de mode de fonctionnement (CAM), comme décrit par exemple par le EP-A-0 488 904 (ELA Médical).

Plus précisément ces dispositifs, qui comportent des moyens de stimulation et de détection à la fois sur l'oreillette et le ventricule, peuvent opérer selon deux modes, DDD ou AAI (le mode AAI étant en fait un mode DDD comprenant un délai atrio-ventriculaire allongé), avec basculement automatique d'un mode à l'autre.

Le mode de fonctionnement de base est un mode AAI, avec une stimulation auriculaire simple chambre. Ce mode est maintenu tant que la conduction atrio-ventriculaire est normale, c'est-à-dire tant que chaque événement auriculaire (détection auriculaire, correspondant à un activité spontanée, ou stimulation auriculaire) est suivi d'une détection ventriculaire associée.

Dans certaines circonstances, notamment des épisodes d'effort, peuvent cependant apparaître des blocs atrio-ventriculaires (BAV), dits "paroxystiques", entraînant un défaut de dépolarisation du ventricule. Dans ce cas, le stimulateur bascule en mode DDD automatique, avec des paramètres optimisés pour cette situation de BAV temporaire. En particulier, pour favoriser le retour d'une conduction atrio-ventriculaire spontanée, le stimulateur applique un délai atrio-ventriculaire (DAV) relativement long, susceptible de laisser s'exprimer la conduction atrio-ventriculaire du patient. Après disparition du BAV, et donc rétablissement de la conduction AV (atrio-ventriculaire) spontanée, le stimulateur retourne automatiquement au mode AAI, dès lors qu'un certain nombre de conditions correspondantes sont remplies.

Ainsi, l'algorithme DDD CAM, pour favoriser la conduction spontanée, la laisse s'exprimer jusqu'à des valeurs de temps de conduction assez longue avant de basculer en stimulation ventriculaire (ici et dans la suite, par "temps de conduction", on entendra l'intervalle PR, c'est-à-dire l'intervalle compris entre une détection atriale et une détection ventriculaire consécutive correspondante, ou bien l'intervalle AR, c'est-à-dire l'intervalle compris entre une stimulation atriale et une détection ventriculaire consécutive correspondante).

Il est en effet souhaitable de laisser s'exprimer la conduction spontanée, généralement préférable du point de vue physiologique (meilleur remplissage des cavités, etc.) à un fonctionnement stimulé.

Le point de départ de l'invention est la constatation que, dans certains cas, il n'est pas toujours souhaitable d'essayer de laisser s'exprimer la conduction AV spontanée du patient.

En effet, il existe certains types de BAV pour lesquels les défauts de conduction AV de certains patients peuvent se traduire par une absence de réduction (voire même un allongement) du temps de conduction PR (ou AR) à l'effort : la conduction existe, mais elle n'est pas bonne hémodynamiquement, et il serait alors préférable de stimuler quand même.

L'invention propose un dispositif perfectionné permettant de diagnostiquer ces situations de mauvaise adaptation à l'effort du temps de conduction, et d'y remédier par une modification appropriée du fonctionnement du stimulateur.

Par ailleurs, le US-A-5 873 895 propose d'adapter le temps de conduction d'un stimulateur asservi en modifiant l'hystérésis du délai AV en fonction du patient. Mais cette adaptation est opérée de façon indifférenciée entre phases d'effort et phases de repos, qui ne sont pas discriminées.

Le type de dispositif auquel s'applique l'invention est un dispositif de type DDD CAM tel que décrit par exemple dans les EP-A-0 488 904 et US-A-5 873 895 précités, et correspondant au préambule de la revendication 1. L'invention est définie par les caractéristiques exposées dans la partie caractérisante de la revendication 1. Les sous-revendications visent diverses formes particulières de mise en oeuvre.

On va maintenant décrire un exemple de mise en oeuvre de l'invention, en référence aux dessins annexés.

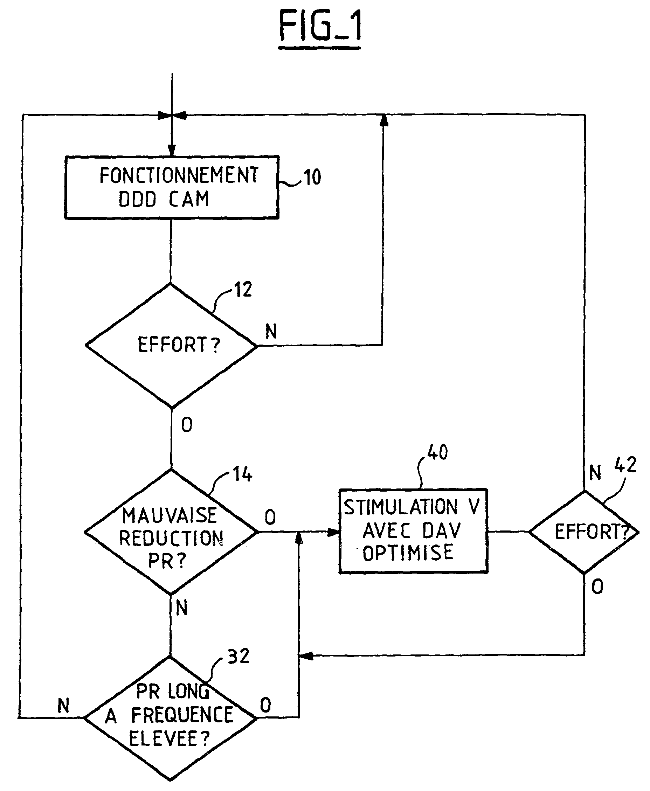

La figure 1 est un organigramme de l'algorithme permettant de mettre en oeuvre les fonctions de l'invention.

La figure 2 illustre les variations du temps de conduction en fonction de la fréquence cardiaque pour diverses situations de bonne ou mauvaise adaptation à l'effort de ce temps de conduction.

L'invention est avantageusement mise en oeuvre par un logiciel intégré à l'algorithme DDD CAM d'un stimulateur cardiaque de type classique. Initialement, le stimulateur, qui a détecté un BAV paroxystique, opère en mode AAI en fonctionnement DDD CAM (étape 10).

Les fonctions mises en oeuvre par le logiciel de l'invention ne le sont que pendant les phases d'effort du patient, phases qui sont détectées (étape 12) au moyen d'un capteur d'activité, permettant de détecter rapidement un changement de l'activité du porteur de l'appareil, typiquement un accéléromètre ("capteur G"), comme enseigné par exemple dans les EP-A-0 550 293 (ELA Médical) ou EP-A-0 750 920 (ELA Médical), ce dernier document décrivant un stimulateur à double capteur, physiologique et d'activité, ou au moyen d'une combinaison de ces deux capteurs.

La première étape consiste à diagnostiquer une mauvaise adaptation du temps de conduction (PR ou AR) à l'effort.

À cet effet, on examine tout d'abord (étape 14) la manière dont varie le temps de conduction en fonction de l'augmentation de la fréquence cardiaque.

Ce diagnostic se fait au début d'un effort, et il n'est opéré que si l'activité sinusale est stable pendant toute la durée du diagnostic, c'est-à-dire si les événements auriculaires sont tous spontanés ou tous stimulés.

Si tel est le cas, le dispositif mémorise en permanence la moyenne des temps de conduction PR (ou AR), par exemple pour quatre bandes de fréquences successives espacées de 10 cpm.

L'analyse de ces données est réalisée de la manière suivante (qui n'a bien entendu aucun caractère limitatif) :

- pour chaque multiple de 10 cpm de la valeur de la fréquence cardiaque entre 50 et 120 cpm, on définit une plage d'intervalles [60000/f - 20 ms ; 60000/f + 20 ms] correspondant à cette fréquence f, par exemple un intervalle [980 ms ; 1 020 ms] pour 60 cpm, un intervalle [580 ms ; 620 ms] pour 100 cpm, etc. ;

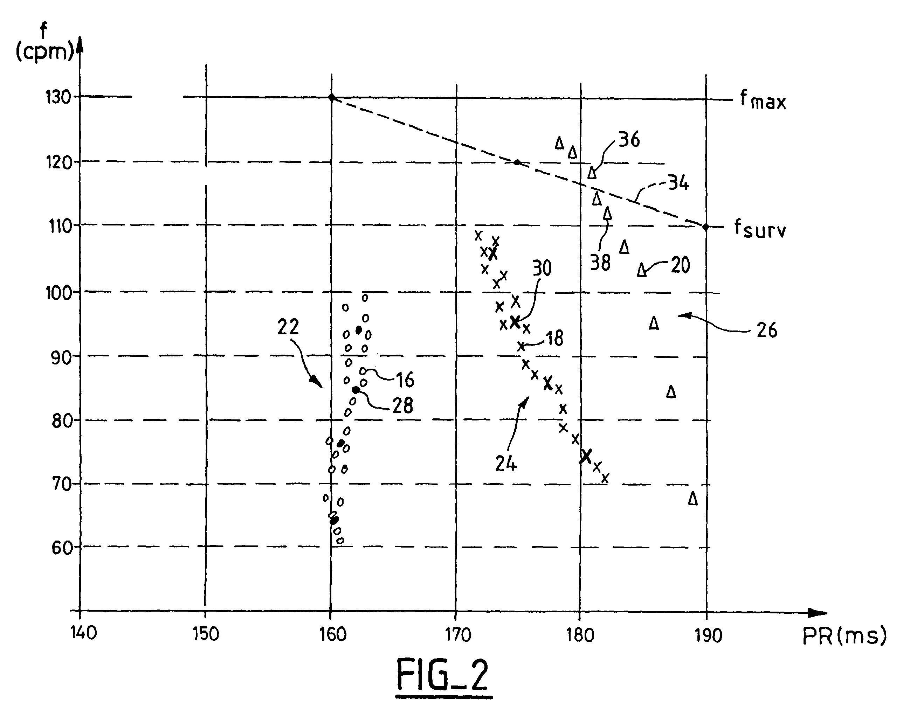

- sur chaque événement atrial (détecté ou stimulé), si l'intervalle atrial qui se termine en l'absence d'élément perturbant (extrasystole auriculaire ou ventriculaire, bruit) et appartient à une plage telle que définie ci-dessus, le dispositif mémorise alors le temps de conduction PR (ou AR) compté à partir de cet événement atrial, lié à la fréquence correspondante ; on peut ainsi mémoriser jusqu'à quatre valeurs de conduction par valeur de fréquence. Ces valeurs ont été portées sur la figure 2, et correspondent aux séries de points tels que 16, 18, 20, respectivement dans trois cas différents de variation du temps de conduction à l'effort (séries 22, 24 et 26) ;

- on obtient ainsi des valeurs de temps de conduction que l'on peut moyenner sur plusieurs mesures, par exemple sur quatre mesures, comme repéré en 28 ou 30 sur la figure 2 (respectivement pour les séries de points 22 et 24). Les valeurs ainsi obtenues pour l'une des fréquences f atteinte au cours de l'effort seront désignées $PRmoy_f$ ;

- le diagnostic est possible lorsque l'on a recueilli un nombre suffisant de valeurs $PRmoy_f$, par exemple des valeurs $PRmoy_f$ pour quatre fréquences successives espacées de 10 cpm ; en variante, on peut également tester la diminution du temps de conduction PR (ou AR) par rapport à un seuil minimal, par exemple une diminution du temps de conduction d'au moins 4 ms pour une augmentation de fréquence de 10 cpm ;

À partir des données ainsi recueillies et classifiées, le dispositif détermine si l'adaptation du temps de conduction PR (ou AR) à l'effort est bonne ou mauvaise.

On peut ainsi considérer que l'adaptation est bonne lorsque le temps de conduction diminue chaque fois qu'augmente la fréquence, plus précisément chaque fois que la fréquence augmente de 10 cpm, c'est-à-dire si l'on a :

$$PRmoy_{fd} > PRmoy_{fd+10} > PRmoy_{fd+20} > PRmoy_{fd+30},$$

fd correspondant à la première valeur de fréquence multiple de 10 cpm, atteinte au début de l'effort. Une hystérésis peut être associée à chacune de ces valeurs.

Le diagnostic est fait une fois par phase d'effort, lorsqu'une augmentation de 30 cpm de la fréquence a été réalisée, et permet de conclure à une bonne ou une mauvaise adaptation du PR à l'effort.

Si l'effort a été trop faible, entraînant une augmentation de la fréquence cardiaque de moins de 30 cpm, ou si l'on n'a pas pu mémoriser de valeur de PR (ou AR) pour chaque valeur de fréquence multiple de 10 cpm, ou encore si l'état stimulé/détecté de l'activité sinusale a changé, alors on considère que le diagnostic d'adaptation du temps de conduction à l'effort est impossible pour cette phase d'effort, et l'on attendra la prochaine phase d'effort pour réitérer l'analyse.

Une autre manière de détecter une mauvaise adaptation du temps de conduction à l'effort consiste, en complément ou en variante de la manière précédente, à examiner la valeur maximale du délai atrio-ventriculaire (DAV) à fréquence élevée.

Ainsi (étape 32), si la fréquence cardiaque courante devient élevée, par exemple dépasse la fréquence maximum programmée $f_{max}$ moins 20 cpm, donnant ainsi la valeur désignée $f_{surv}$ (fréquence de surveillance) sur la figure 2, on considère que, si l'adaptation à l'effort est bonne, le DAV ne doit pas dépasser une valeur donnée, fixée soit de manière absolue (typiquement 180 ms), soit en pourcentage de la durée du cycle cardiaque (typiquement 35 %, limite repérée graphiquement en 34 sur la figure 2).

On examine ainsi au cours de cycles successifs si le temps de conduction dépasse (comme en 36) ou non (comme en 38) la limite ainsi fixée. Si cette limite est, par exemple, dépassée sur quatre cycles successifs, ceci signifie que le temps de conduction (PR ou AR) est plus long que la valeur maximale prévue, et le dispositif conclut donc à une mauvaise adaptation à l'effort.

Lorsqu'une mauvaise adaptation à l'effort est diagnostiquée par l'une et/ou l'autre des méthodes ci-dessus, l'action décidée par le dispositif consiste (étape 40) :

- d'une part, à désactiver la commutation automatique de mode du stimulateur, c'est-à-dire à ne plus rechercher une activité spontanée (donc ne plus chercher à retourner au mode AAI pendant toute la durée de l'effort) et maintenir le stimulateur sur un mode DDD simple (sans CAM), et

- d'autre part et surtout, à reprogrammer le DAV à une valeur plus courte, optimisée pour la stimulation permanente pendant l'effort.

La recherche de conduction spontanée (impliquant donc l'application d'un DAV plus long) ne sera reprise que lorsque l'effort sera terminé, c'est-à-dire quand le capteur d'activité aura indiqué un retour à l'état de repos (étape 42).

**Revendications**

1. Un dispositif médical implantable actif, notamment un stimulateur cardiaque, défibrillateur et/ou cardioverteur de type DDD/AAI à commutation automatique de mode, comprenant :

   - des moyens de recueil des événements auriculaires et ventriculaires spontanés,
   - des moyens de détection de la présence ou de l'absence d'une conduction spontanée atrio-ventriculaire,
   - des moyens de stimulation ventriculaire et auriculaire, la stimulation ventriculaire étant appliquée, sur détection de l'absence de conduction spontanée atrio-ventriculaire, après écoulement d'un délai atrio-ventriculaire programmé déclenché sur un événement auriculaire, spontané ou stimulé, et
   - des moyens de mesure du temps de conduction atrio-ventriculaire séparant un événement auriculaire, spontané ou stimulé, d'un événement ventriculaire spontané consécutif correspondant, et
   - des moyens pour discriminer (12, 42) entre phases d'effort et de repos du porteur du dispositif, dispositif **caractérisé en ce qu'**il comprend en outre :

     - des moyens (14, 32) de diagnostic de bonne ou mauvaise adaptation du temps de conduction à l'effort, ces moyens opérant par évaluation, pendant une phase d'effort, de la variation du temps de conduction en fonction de la variation de la fréquence cardiaque.

2. Le dispositif de la revendication 1, comprenant en outre des moyens (40) aptes, lorsque les moyens de diagnostic indiquent une mauvaise adaptation à l'effort, à désactiver la commutation automatique de mode du dispositif.

3. Le dispositif de la revendication 1, comprenant en outre des moyens (40) aptes, lorsque les moyens de diagnostic indiquent une mauvaise adaptation à l'effort, à basculer le dispositif en mode DDD.

4. Le dispositif de la revendication 1, comprenant en outre des moyens (40) aptes, lorsque les moyens de diagnostic indiquent une mauvaise adaptation à l'effort, à reprogrammer le délai atrio-ventriculaire à une valeur plus courte que la valeur antérieurement programmée.

5. Le dispositif de la revendication 1, dans lequel les moyens pour discriminer entre phases d'effort et de repos comportent un capteur d'activité.

6. Le dispositif de la revendication 1, dans lequel les moyens de diagnostic ne sont mis en oeuvre que si, pendant la durée de l'évaluation, les événements auriculaires sont tous des événements spontanés ou bien tous des événements stimulés.

7. Le dispositif de la revendication 1, dans lequel les moyens de diagnostic déterminent qu'il y a mauvaise adaptation à l'effort lorsque ladite évaluation révèle une absence de réduction du temps de conduction pour une augmentation de la fréquence cardiaque.

8. Le dispositif de la revendication 7, dans lequel ladite évaluation est opérée en mémorisant des valeurs successives (16 ; 18 ; 20) du temps de conduction, ou des moyennes (28 ; 30) de ces valeurs, relevées pour une pluralité de valeurs successives prédéterminées de la fréquence cardiaque.

9. Le dispositif de la revendication 8, dans lequel les valeurs du temps de conduction mesuré ne sont retenues pour l'évaluation que lorsqu'elles sont comprises à l'intérieur d'un intervalle prédéfini autour de la fréquence cardiaque prédéterminée correspondante.

10. Le dispositif de la revendication 1, dans lequel les moyens de diagnostic ne sont mis en oeuvre que si l'augmentation de la fréquence cardiaque depuis le début de la phase d'effort est supérieure à un seuil prédéterminé.

11. Le dispositif de la revendication 1, comprenant en outre des moyens aptes à limiter (34) la valeur du délai atrio-ventriculaire en phase d'effort lorsque la fréquence cardiaque ($f_{surv}$) devient supérieure à un seuil prédéterminé.

12. Le dispositif de la revendication 1, dans lequel les moyens de diagnostic déterminent qu'il y a mauvaise adaptation à l'effort lorsque, sur un nombre prédéterminé de cycles cardiaques présentant une fréquence supérieure à une fréquence cardiaque prédéterminée, le temps de conduction est supérieur à une durée maximale prédéterminée.

13. Le dispositif de la revendication 1, dans lequel la désactivation de la commutation automatique de mode, le basculement en mode DDD et la reprogrammation du délai atrio-ventriculaire sont opérés pendant toute la durée de la phase d'effort, le fonctionnement antérieur n'étant rétabli qu'après retour à une phase de repos.

**Patentansprüche**

1. Aktive implantierbare medizinische Vorrichtung, insbesondere Herzschrittmacher, Defibrillator und/oder Kardioverter vom DDD/AAI-Typ mit automatischer Modus-Umschaltung, umfassend:

   - Mittel zum Empfang von spontanen atrialen und ventrikulären Ereignissen,
   - Mittel zur Detektion des Vorhandenseins oder der Abwesenheit einer spontanen atrio-ventrikulären Leitung,
   - Mittel zur ventrikulären und atrialen Stimulation, wobei die ventrikuläre Stimulation bei Detektion der Abwesenheit von spontaner atrio-ventrikulärer Leitung, nach Ablauf einer programmierten atrio-ventrikulären Verzögerung, ausgelöst bei einem atrialen Ereignis, spontan oder stimuliert angewandt wird, und
   - Mittel zur Messung der Dauer der atrio-ventrikulären Leitung, die ein atriales Ereignis, spontan oder stimuliert, von einem entsprechenden folgenden spontanen ventrikulären Ereignis trennt,
   - Mittel zum Unterscheiden (12, 42) zwischen Phasen der Belastung und der Ruhe des Trägers der Vorrichtung, wobei die Vorrichtung **dadurch gekennzeichnet ist, dass** sie ferner umfasst:

     - Mittel (14, 32) zur Diagnose der guten oder schlechten Anpassung der Leitungsdauer an die Belastung, wobei diese Mittel während einer Belastungsphase mit einer Berechnung der Variation der Leitungsdauer abhängig von der Variation der Herzfrequenz arbeiten.

2. Vorrichtung nach Anspruch 1, ferner umfassend Mittel (40), die dazu geeignet sind, die automatische Umschaltung des Modus der Vorrichtung zu deaktivieren, wenn die Mittel zur Diagnose eine schlechte Anpassung an die Belastung anzeigen.

3. Vorrichtung nach Anspruch 1, ferner umfassend Mittel (40), dazu geeignet, die Vorrichtung in den DDD-Modus umzuschalten, wenn die Mittel zur Diagnose eine schlechte Anpassung an die Belastung anzeigen.

4. Vorrichtung nach Anspruch 1, ferner umfassend Mittel (40), dazu geeignet, die atrio-ventrikuläre Verzögerung auf einen kürzeren Wert zu reprogrammieren als den zuvor programmierten Wert, wenn die Mittel zur Diagnose eine schlechte Anpassung an die Belastung anzeigen.

5. Vorrichtung nach Anspruch 1, bei welcher die Mittel zum Unterscheiden zwischen Belastungs- und Ruhephasen einen Aktivitätssensor aufweisen.

6. Vorrichtung nach Anspruch 1, bei welcher die Mittel zur Diagnose nicht in Betrieb gesetzt werden, außer wenn während der Dauer der Berechnung die atrialen Ereignisse alle spontane Ereignisse sind oder auch alle stimulierte Ereignisse sind.

7. Vorrichtung nach Anspruch 1, bei welcher die Mittel zur Diagnose bestimmen, ob eine schlechte Anpassung an die Belastung vorliegt, wenn die Berechnung eine Abwesenheit einer Reduktion der Leitungsdauer für eine Erhöhung der Herzfrequenz ergibt.

8. Vorrichtung nach Anspruch 7, bei welcher die Berechnung ausgeführt wird durch Speichern aufeinanderfolgender Werte (16; 18; 20) der Leitungsdauern oder Mittelwerte (28; 30) dieser Werte, erhoben für eine Vielzahl von vorherbestimmten aufeinanderfolgenden Werten der Herzfrequenz.

9. Vorrichtung nach Anspruch 8, bei welcher die Werte der gemessenen Leitungsdauer nicht für die Berechnung aufbewahrt werden, es sei denn sie sind in einem vordefinierten Wertebereich, um die entsprechende vorherbestimmte Herzfrequenz enthalten.

10. Vorrichtung nach Anspruch 1, bei welcher die Mittel zur Diagnose nicht in Betrieb gesetzt werden, außer wenn die Erhöhung der Herzfrequenz seit dem Beginn der Anstrengungsphase größer ist als ein vorherbestimmter Schwellwert.

11. Vorrichtung nach Anspruch 1, ferner umfassend Mittel, die geeignet sind, den Wert der atrio-ventrikulären Verzögerung in der Anstrengungsphase zu beschränken (34), wenn die Herzfrequenz ($f_{surv}$) größer wird als ein vorherbestimmter Schwellwert.

12. Vorrichtung nach Anspruch 1, bei welcher die Mittel zur Diagnose bestimmen, ob eine schlechte Anpassung an die Belastung vorliegt, wenn, über eine vorherbestimmte Anzahl von Herzzyklen, die eine Frequenz größer als eine vorherbestimmte Herzfrequenz aufweisen, die Leitungsdauer größer als eine vorherbestimmte maximale Dauer ist.

13. Vorrichtung nach Anspruch 1, bei welcher die Deaktivierung der automatischen Umschaltung des Modus, das Umschalten in den DDD-Modus und die Reprogrammierung der atrio-ventrikulären Verzögerung während der gesamten Dauer der Belastungsphase durchgeführt werden, wobei das vorhergehende Funktionieren nur nach Rückkehr zu einer Ruhephase wiederhergestellt wird.

## Claims

1. An active implantable medical device, in particular a cardiac pacemaker, defibrillator and/or cardioverter of the DDD/AAI type and automatic mode switching including:

   - means for sensing spontaneous atrial and ventricular events,
   - means for detecting the presence or the absence of a spontaneous atrio-ventricular conduction,
   - means for ventricular and atrial stimulation, the ventricular stimulation being applied, upon detection of the absence of spontaneous atrioventricular conduction, following a programmed atria-ventricular delay triggered on an atrial event, either stimulated or spontaneous, and
   - means for measuring the atrio-ventricular conduction time separating an atrial event, either stimulated or spontaneous, from a consecutive corresponding spontaneous ventricular event, and
   - means for discriminating (12, 42) between effort and rest phases of the bearer of the device,

   said device being **characterised by** further comprising:

   - means (14, 32) for diagnosing either good or bad adaptation to effort of the conduction time, said means operating by evaluating, during an effort phase, the variation of the conduction time as a function of the variation of the heart rate.

2. The device of claim 1, further comprising means (40) adapted, when the diagnosis means indicates a bad adaptation to effort, to deactivate the automatic mode switching of the device.

3. The device of claim 1, further comprising means (40) adapted, when the diagnosis means indicates a bad adaptation to effort, to switch the device to DDD mode.

4. The device of claim 1, further comprising means (40) adapted, when the diagnosis means indicates a bad adaptation to effort, to reprogram the atrioventricular delay to a value shorter than the previously programmed value.

5. The device of claim 1, wherein the means for discriminating between effort and rest phases comprises an activity sensor.

6. The device of claim 1, wherein the diagnosis means is enabled only if, for the duration of the evaluation, the atrial events are either all spontaneous events or all stimulated events.

7. The device of claim 1, wherein the diagnosis means determines that there is a bad adaptation to effort when said evaluation indicates an absence of a reduction of the conduction time for an increase in the heart rate.

8. The device of claim 7, wherein said evaluation is performed by storing successive values (16 ; 18 ; 20) of the conduction time, or averages (28 ; 30) of said values, recorded for a plurality of predetermined successive values of the heart rate.

9. The device of claim 8, wherein the values of the conduction time measured are retained for evaluation only if they are included inside a predefined interval around the corresponding predetermined heart rate.

10. The device of claim 1, wherein the diagnosis means are enabled only if the increase in the heart rate since the beginning of the effort phase is higher than a predetermined threshold.

11. The device of claim 1, further comprising means adapted to limit (34) the value of the atria-ventricular delay in an effort phase when the heart rate ($f_{surv}$) becomes higher than a predetermined threshold.

12. The device of claim 1, wherein the diagnosis means determines that there is a bad adaptation to effort when, for a predetermined number of cardiac cycles presenting a rate higher than a predetermined heart rate, the conduction time is longer than a predetermined maximum duration.

13. The device of claim 1, wherein the deactivation of the automatic mode switching, the switching to the DDD mode and the reprogramming of the atrio-ventricular delay are performed during the whole duration of the effort phase, the prior operation being restored only after the return to a rest phase.

# FIG_1

FIG_2